Europäisches Patentamt

European Patent Office

Office européen des brevets

(11) Publication number: **0 190 926 B1**

## EUROPEAN PATENT SPECIFICATION

(45) Date of publication of patent specification: **10.04.91**   (51) Int. Cl.⁵: **A61K  9/50**

(21) Application number: **86300775.3**

(22) Date of filing: **05.02.86**

(54) **A method for the preparation of liposomes, a pack for use therein and a composition for use in preparing a liposomal material.**

(30) Priority: **05.02.85 GB 8502892**

(43) Date of publication of application:
**13.08.86 Bulletin  86/33**

(45) Publication of the grant of the patent:
**10.04.91 Bulletin  91/15**

(84) Designated Contracting States:
**AT BE CH DE FR IT LI LU NL SE**

(56) References cited:
**EP-A- 0 158 441**
**FR-A- 2 550 706**

**CHEMICAL ABSTRACTS, vol. 97, no. 10, September 1982, page 414, no. 78826w, Columbus, Ohio, US; W.D. SEUFERT et al.: "Dipalmitoylphosphatidylcholine dissolved in ethanol forms highly elastic monolayers" & CAN. J. PHYSIOL. PHARMACOL. 1982, 60(6), 862-4**

(73) Proprietor: **STERWIN AG.**
**Zeughausgasse 9**
**CH-6300 Zug(CH)**

(72) Inventor: **McGurk, John Gerard**
**76 Chapel Lands**
**Alnwick Northumberland NE66 1ER(GB)**

(74) Representative: **Green, Alan James et al**
**SANDERSON & CO. European Patent Attorneys 34, East Stockwell Street**
**Colchester Essex CO1 1ST(GB)**

## Description

The present invention relates to aerosol compositions and, in particular, to a method for the preparation of liposomes, a pack for use therein and a composition for use in preparing a liposomal material. More especially, the invention concerns the preparation of compositions comprising an active material and a liposomal carrier using a pressurised aerosol system.

Certain therapeutically-active compounds are administered to patients by inhalation therapy, that is they are administered to the respiratory tract through the nose or mouth as a vapour or gas, or as a mist together with a carrier material in the form of a vapour or gas. Similarly, in certain treatments of the skin, for example, for the treatment of open wounds or sprains, for the treatment of dermatological conditions, or for cosmetic purposes, it may be desirable to administer an active compound by spraying as a gas or vapour or mist. In either event, it is desirable that as high a proportion as possible of the intact active compound should reach the intended site of action, and sometimes then that active compound should for a time remain intact at the intended site of action. That is to say that the active compound should be administered without breaking down either in transit or immediately on contact with the patient.

Thus, for example, in the administration of the anti-asthmatic bronchodilator compound known as bitolterol (disclosed in British Specification No. 1,298,771) it is desirable to administer the compound in such a manner as to obtain maximum penetration of the bronchi, thereby to obtain the most effective levels of active compound at the necessary sites of action.

In the past, it has been proposed to produce liposome compositions containing biologically-active compounds and such compositions are disclosed, for example, in British Specifications Nos. 1,575,343, 1,575,344 and 2,013,609 A. However, in such known compositions the liposomal material is one which is pre-prepared and dried, typically by lyophilisation (freeze-drying). The liposomal material is then reconstituted by mixing with water.

Also, aerosol compositions containing a variety of cosmetic and therapeutic materials are known - see, for example, British Specifications Nos. 780,885, 993,702, 1,302,671, 1,381,184, 1,516,195 and 2,001,334 A. However, while such known aerosol compositions may contain lipid ingredients, those ingredients are invariably present merely as dispersing, suspending, emulsifying or like agents. Furthermore, such compositions are intended to produce either a dry spray, so that no liposomal formation is possible or, as in the case of the foundation cream of Specification No. 780,885, are presented as an aqueous emulsion in which the function of any lipid which may be present is solely that of an emulsifying or like agent. Similarly, as described in British Specifications Nos. 1,281,696 and 1,352,062, lecithin may be used in very small amounts in photographic processing as part of an aerosol developer formulation containing water to separate the propellant from the developer solution.

In addition, as disclosed in U.S. Specification No. 3,594,476, it has been proposed to prepare lecithin aerosols useful for the treatment of lung disorders, which optionally may contain other therapeutic agents such as antibiotics. In the disclosed method of preparation, there is first prepared a suspension of DL dipalmitoyl-$\alpha$-lecithin in water (or saline solution), with any medicament being dissolved in the water or saline, that suspension then being nebulized i.e. formed into an aerosol, in an ultrasonic generator supplied with a carrier air stream. Thus, the disclosed method relies on relatively expensive ultrasonic equipment to produce the necessary final mixture of lecithin and water, as well as on the use of an initial mixture comprising lecithin suspended in water. Also, ultrasonic equipment can be noisy and would be unsuitable for use on a general basis.

Furthermore, as disclosed in U.S. Specification No. 4,371,451, it has been proposed to produce lecithin-based cookware surface release compositions comprising water, lecithin and dimethylether as propellant. While in that disclosure the compositions are dispensed from an aerosol container, what is sought is a quick-drying and thin coating of lecithin which does not foam, rather than a liposomal material as such. Again, the compositions dispensed for that purpose are produced from an initial mixture comprising a suspension of solid lecithin in an aqueous carrier, that is lecithin dispersed in a solution of dimethylether and water, or in a solution of dimethylether and aqueous ethanol. Similarly, cookware lubricants containing lecithin are disclosed in British Specification No. 1,463,964. However, in that disclosure the lecithin is mixed with relatively large quantities of oily materials to provide a water-in-oil emulsion and sufficient to prevent lipsome formation.

In our co-pending Application No. 84-20850 published as GB 2,145,107A on 20th March, 1985, we have described and claimed a method for the preparation of liposomes, which method comprises bringing together under pressure at least two separate components, a first component comprising water and a second component comprising a lipid material, and passing the mixture under pressure through a nozzle or other arrangement thereby to produce an aerosol spray containing liposomes.

2

In that earlier Application, the claimed method provides for the in situ or extemporaneous preparation of liposomes using a pressurised aerosol system. To achieve that, an aqueous first component and a second component comprising dry lipid material, which are initially kept separate, are mixed in the act of spraying to produce a spray in which the liposomes may be present as such or as a liposomal carrier for one or more other active materials, generally a biologically-active material and/or a material useful in the treatment or care of the human or animal body.

The method of the earlier invention has the advantage that active material and water, and/or propellant and water, essentially can be kept separate until dispensing of each required dose takes place. As will be appreciated, that separation of ingredients which potentially can interact with consequent degradation, can lead to the provision of a shelf life of sufficient length to enable the method to be applied within a wide variety of practical contexts. At the same time, however, there may be certain contexts within which the consequent long shelf life may not be required or necessary.

We have now found surprisingly that a much simpler system for the extemporaneous preparation of liposomes may be provided, for example, for use in such contexts, by dissolving a lipid component in an organic solvent, and initially combining the thus-prepared solution with water to provide a reservoir of combined material. The combination comprising a multiple-dose system can be suitably mixed either before being packaged and/or otherwise prior to dispensing, for example, to emulsify the solution and water, and portions of the combination as said mixture discharged through an orifice e.g. in a spray nozzle, to form discrete doses as an aerosol spray containing liposomes.

Accordingly, the present invention provides a method for the preparation of liposomes, which method comprises preparing a solution of a lipid component in an organic solvent, combining the thus-formed solution with a second component comprising water, forming an intimate mixture from the combination, and passing a portion of the combination as said mixture under pressure through an orifice e.g. in a spray nozzle or other arrangement, thereby to produce an aerosol spray containing liposomes.

As with the method of our earlier Application, the method of the present invention provides for the extemporaneous preparation of liposomes using a pressurised aerosol system. However, in the present method, an aqueous component and a solution of a lipid component in an organic solvent are first combined to form a reservoir of combined material which affords a multiple-dose system (for example, they are pre-packaged together), the system then being subsequently employed to form a mixture, typically as an aqueous emulsion, which can be passed through an orifice under pressure to produce discrete doses as a spray in which liposomes may be present as such or as a liposomal carrier for one or more other materials. Thus again, in one preferred embodiment of the present invention there is produced a liposomal aerosol including liposomal carrier and a separate active material which generally will be one which is biologically-active and/or useful in the treatment or care of the human or animal body or of plants. Such an active material also will generally be a non-lipid material, although in some instances a lipid may be used to carry an active moiety, for example, attached thereto.

Alternatively, the invention can be used to prepare and provide a liposomal aerosol as such, that is without a separate active compound or other material. Again, such aerosols would find application in a number of ways, either to prepare liposomes per se for a variety of applications, or in therapy. In particular, they would be useful in parturition where newborn infants with breathing difficulties (respiratory distress syndrome) could be assisted to breathe more normally by administering a lipid material to their bronchial regions to supplement the low levels of naturally present lung surfactant found in these infants.

Preferably, however, a separate active material is provided, which initially may be present either in solution in the aqueous component or in solution in the organic solvent. On the other hand, whether or not a separate active material is provided, in each case a combination of the lipid solution and the aqueous component, which generally represents a system comprising more than a single dose of active material, is employed in the dispensing of single doses as a spray.

In the method of the invention, the action of spraying the said intimate mixture leads to the preparation of a liposomal material. Typically, the mixing step will produce a mixture which either as such or during spraying will comprise an aqueous emulsion containing lipid micelles from which the liposomal material can form. In that respect, and while we do not wish the present invention to be limited to any one particular hypothesis, we believe the formation of liposomes is accomplished on spraying by the disruption of the initially formed lipid micelle-containing aqueous emulsion when the organic solvent evaporates during spraying and the stabilising effect which it exerts on the lipid phase is reduced or eliminated. Single wall micelles in the emulsion can then open and thus-formed lipid layers can fasten themselves around other intact micelles to form double- walled material, i.e. a liposome.

Furthermore, where an active material is present the liposomal material includes active material carried by an aqueous medium either trapped in encapsulated form between the lipid layers or sandwiched between molecules of lipid. By varying the conditions under which the mixture is formed and/or sprayed, the size of the liposomes and the relative proportions of free and incorporated active material can be varied within wide limits, the latter up to about 100% incorporation. For example, by forming and/or spraying the aerosol mixture under varying degrees of mixing activity, for example, as provided by simple shaking together of the combined components, or by turbulent conditions created say in a mixing chamber and/or in feed means associated therewith and/or in a spray nozzle, a spray can be formed either as one having a high proportion of free active material-giving fast action with a reserve of slow-release active material - or as one having a lower proportion of free active material - giving a slower action with a more prolonged and controlled release of active material. Thus, in the latter case, there may be produced a composition comprising active material in which a high proportion of the material is enabled to be carried in a "protected" form to the intended site of action, and/or otherwise to act in a sustained-release manner.

The method of the invention is applicable to the preparation of compositions comprising a wide variety of biologically-active materials and/or other materials useful in the treatment or care of the human or animal body or of plants. Typically, the method may be employed to prepare compositions comprising therapeutically-active compounds, including those used in veterinary treatments, as well as biologically-active reagents, cosmetically-active materials, compounds having nutritional value, and other materials used to treat or care for the human or animal body. Also, the compositions may include such plant treatment agents as herbicides, pesticides, plant growth regulators and the like.

Suitable cosmetically-active materials include products intended for skin care and hair care, for example, humectants, artificial tanning agents (optionally in association with colourants), anti-sunburn agents, antiperspirants, deodorants, astringents and freshening, toning, cicatrisant, keratolytic and depilatory products, perfumed water, extracts of animal or plant tissues, hair dyes, anti-dandruff agents, antiseborrhoea agents, oxidising agents e.g. bleaching agents, and reducing agents.

Therapeutically-active compounds which may be mentioned include vitamins, steroids, e.g. corticosteroids, hormones, active peptides, enzymes, vaccines, anti-inflammatory agents, antibiotics and bactericides. However, the method of the invention is particularly applicable to the preparation of liposomes which may be used in the administration of therapeutically-active compounds used in inhalation therapy much as bronchodilators and anti-asthmatic compounds, as well as anti-tumour agents. Such compounds included in or with the liposomal carrier are able to penetrate the bronchial tree, thus enabling the effectiveness of the therapy to be advantageously modified. Similarly, the method has particular advantages in the preparation of liposomes which may be used in the treatment of open wounds, e.g. ulcerated wounds, where the liposomal carrier also can be employed to produce (to advantage) a modified activity, enabling treatment essentially to be confined to the wound itself without any pronounced systemic dispersion.

In accordance with the above, and by way of example only, the following specific compounds and routes of administration may be mentioned:

| Active Compound | Class of Compound | Route of Administration |
|---|---|---|
| Stanozolol | Anabolic Steroid | Topical (e.g. open wound treatment) |
| Hydrocortisone (and its esters) | Anti-inflammatory steroid | Topical |
| Betamethasone (and its esters e.g. its valerate) | Anti-inflammatory steroid | Topical |
| Bitolterol (and its salts e.g. its mesylate) | Catecholamine bronchodilator | Inhalation |
| Salbutamol | Catecholamine bronchodilator | Inhalation |
| Theophylline | Xanthene bronchodilator | Inhalation |
| Sodium chromoglycate | Non-steroidal anti-asthmatic | Inhalation |
| N-Acetylmuramyl-L-alanyl-D-isoglutamine (adjuvant dipeptide) | Macrophage activating agent (Anti-tumour dipeptide) | Inhalation/ intranasal |
| Propranolol | B-blocking agent and contraceptive | Oral/ Intravaginal |

Of course, it will be understood by those skilled in the art that a wide variety of other active materials may be employed. Furthermore, while topical, inhalation, intranasal, and oral routes may be most frequently employed, other routes of administration may be used as appropriate. For example, compositions produced in the method of the invention containing appropriate active materials could be administered direct say to the eye or even intravenously.

In addition, there may be mentioned biologically-active materials which may also be therapeutically active such as vaccines, or other biologically-active materials useful as reagents such as antigens and/or antibodies e.g. anti-rabbit IgG. Reagents of this kind may be useful in say testing blood samples, typically for the presence or absence of disease.

The liposomal material produced in the invention may be one which is positively or negatively charged or one which is neutral. The material comprises lipid material existing in aqueous dispersion or suspension, and may include one or more other active materials. Furthermore, the lipid material may be in the form of unilamellar and/or multilamellar lipid bilayers which may carry associated with them any said other active material.

The liposomal material typically may comprise any amphipathic lipid which is suitable for the preparation of liposomes and generally conforming to the formula:

X - Y

where X is a polar hydrophilic group and Y is a non-polar hydrophobic group.

Preferably, however, the liposomal material comprises a phospholipid, and more preferably is a phospholipid which comprises a phosphatidyl choline. For example, a dipalmitoyl phosphatidyl choline as such or in the form of lecithin; a phosphatidyl ethanolamine; a phosphatidyl serine; a phosphatidyl sphingomyelin; a phosphatidyl cardiolipin; a phosphatidyl plasmalogen; phosphatidic acid; and/or a phosphatidic cerebroside may be employed as lipid material. Also, the liposomal material may be formed from a phospholipid e.g. lecithin, alone or together with cholesterol and/or at least one compound which can

5

confer charge on the liposomal material. Thus, for example, charge may be conferred on the liposomal material by employing dicetyl phosphate or phosphatidic acid, which can give a negatively charged lipid material, or stearylamine, which can give a positively charged lipid material.

The use of the above materials and their proportions, as well as the wide choice of materials and the variations in proportions which may be employed, is well known to the man skilled in the art. Generally speaking a wide range of ratios of lipid materials will afford the required liposomal material, provided the lipid component generally comprises no more than say about 50% by weight of cholesterol and/or no more than say about 20% by weight of a material to confer charge.

However, by way of example only, in one preferred embodiment in accordance with the invention, the lipid component may comprise a phosphatidyl choline and cholesterol, typically in relative molar proportions of about 8 : from about 1 to about 2, e.g. about 8.5 : 1.

In another preferred embodiment in accordance with the invention, the lipid component may comprise a phosphatidyl choline, cholesterol and dicetyl phosphate, typically in relative molar proportions of about 8 : from about 1 to about 2 : from about 1 to about 0.05, e.g. about 8.5 : 1 : from about 0.7 to about 0.07.

In a further preferred embodiment in accordance with the invention, the lipid component may comprise a phosphatidyl choline, cholesterol and phosphatidic acid, typically in relative molar proportions of about 8 : from about 1 to about 2 : from about 1 to about 0.1 e.g. about 8.5 : 1 : about 0.1.

In yet another preferred embodiment in accordance with the invention, the lipid component may comprise a phosphatidyl choline, cholesterol and stearylamine, typically in relative molar proportions of about 8 : from about 1 to about 2 : from about 1 to about 0.05, e.g. about 8.5 : 1 : about 0.06.

Alternatively, the lipid component may comprise, for example:

a mixture of a phosphatidyl choline e.g. lecithin, and phosphatidic acid e.g. in a ratio of about 60 : about 1;

a mixture of a phosphatidyl choline e.g. lecithin, and dicetyl phosphate e.g. in a ratio of about 15 : about 1; or, most preferably,

lecithin itself.

The liposomal material produced in the invention may have a pH value within the physiological range, and thus preferably in the range of from about 6.8 to about 7.4, more preferably of from about 7 to about 7.4. To that end the system comprising said solution and said aqueous component may also contain a buffering material in an amount appropriate to establish and maintain the desired pH, and suitable for that purpose is a phosphate buffer, which typically may comprise sodium dihydrogen phosphate and disodium hydrogen phosphate. However, in some instances e.g. where the active material comprises certain active compounds, or a reagent such as a test marker, the pH may be outside the physiological range. Thus, for example, in the case of hydrocortisone the pH may have to be a pH less than about 5 for stability reasons.

The organic solvent employed in the invention may be any solvent which dissolves the chosen lipid component, and which is otherwise acceptable within the desired use context. Preferably, however, the solvent is one which is physiologically-acceptable, and one in which both the lipid component and any other active material are soluble. In that respect, preferred solvents are relatively volatile organic solvents, for example, alcohols such as ethanol and isopropyl alcohol, chloroform (subject to local regulations) and ether. Also, the second component comprising water may be formed as one containing a small proportion of a compatible organic solvent, such as ethanol, for example, in an amount up to about 10% by weight.

In a preferred embodiment of the invention, the aerosol spray is produced by the use of one or more aerosol propellants. Such a propellant or propellants may be included in the system comprising said solution and said aqueous component and/or supplied separately. Preferably, however, a single system comprising one or more propellants, said solution and said aqueous component is provided.

Typically, the propellant may comprise a hydrocarbon or halogenated hydrocarbon or mixture of such hydrocarbons. Examples of suitable propellants are propane, butane, compressed air, nitrogen, dimethylether, and propellant 11 (trichlorofluoromethane), although dichlorodifluoromethane or dichlorotetrafluoroethane or mixtures thereof are preferred. The propellant may be one formulated as a low pressure propellant developing pressures up to say from about 25 to about 30 psi such as a 20:80 w/w mixture of dichlorodifluoromethane and dichlorotetrafluoroethane. Alternatively, the propellant may be a relatively high pressure propellant developing pressures up to say about 70 psi such as 80:20 w/w mixture of dichlorodifluoromethane and dichlorotetrafluoroethane.

In the method of the invention, the chosen composition may be dispensed using a variety of standard spray valve arrangements. However, where the composition includes an active material which it is desired to dispense in controlled discrete doses, the composition should preferably be discharged through an orifice under pressure via a dosing valve which affords the desired metered dose of any said active material for each valve actuation.

Also, in the method of the invention, the emulsion should contain enough water sufficiently to hydrate the lipid. Thus, although water to lipid ratios down to about 2:1 on a weight basis may be employed, it is preferred to employ a weight ratio of water to lipid of at least about 5:1, more preferably at least about 10:1, for example, about 20:1 or higher e.g. up to about 30:1. In addition, there must be employed a sufficient amount of organic solvent to dissolve the lipid component so as to avoid the production of an initial dispersion of solid lipid particles in water. Also, the amount of water present in the mixture should generally exceed the amount of propellant (if mixed therewith), and preferably the water is i n an excess of at least about 1.5:1 over any propellant mixed therewith. Furthermore, any materials present in the emulsion and/or their amounts must not be such as will interfere with the ability of any chosen water/lipid mixture to form liposomes.

Accordingly, preferred ranges of amounts by weight hased on the total system and preferred weight ratios for the various ingredients may be illustrated as follows:

Water - at least about 45% and up to about 97%, more preferably from about 45% to about 70%;

Lipid - no more than about 25%, more preferably from about about 2% to about 10%;

Propellant - from about 5% to about 50%, more preferably from about 20% to about 45%;

Solvent - from about 4% to about 20%;

Water/lipid - from about 2:1, more preferably from about 5:1 to about 30:1;

Solvent/lipid - about 40:1 or lower e.g. 2:1 or lower;

Water/solution - at least about 1:1, more preferably at least about 4:1;

Water/propellant - from about 1.1:1 to about 20:1;

Propellant/solvent - from about 1.3:1 to about 12:1.

The invention also includes a pack for use in preparing a liposomal aerosol, which pack comprises at least a first chamber containing a solution of a lipid component in an organic solvent and an aqueous component, the first chamber and/or a second chamber including at least one propellant material, and the pack including an arrangement for dispensing through an orifice a mixture comprising the lipid solution and aqueous component as a spray under pressure developed by the propellant material or materials.

Preferably, the pack includes an active material as defined above in solution in the organic solvent or in the aqueous component e.g. in the water thereof. Preferably also, the dispensing arrangement is such that a metered dose of any said active material can be dispensed from the pack, although a continuous flow valve may be employed if desired. Generally, the active material, the lipid solution and aqueous component, and the propellant are as described above for the method of the invention.

In the pack of the invention, the first chamber may be an aerosol can or bag, and any second chamber may be a second aerosol can or bag. Also, where there are two chambers they preferably may be arranged with the first within the second (or vice versa), the valve means being disposed within or above the outer can or other chamber. More preferably, however, the pack of the invention comprises either a single cannister including all of the necessary ingredients and a propellant, or a cannister having an inner flexible bag containing the necessary ingredients, with the propellant in the space between the bag and the cannister. In that latter case the ratio of water to propellant generally may be towards the about 20:1 upper limit given above, since much less propellant is usually required in a two-chamber pack.

Alternatively, in a modification, the pack of the invention may comprise at least a first chamber containing a solution of a lipid component in an organic solvent, means to supply an aqueous component to the chamber, the first chamber, a second chamber and/or the aqueous component including at least one propellant material, and the pack including an arrangement for dispensing through an orifice a mixture of said solution and aqueous component as a spray under pressure developed by the propellant material or materials.

Such a modified pack can be used to produce a pack charged both with the said solution and with the aqueous component at the point of dispensing of the pack to the patient. In that manner the water and lipid may be kept separate until the pack is dispensed, e.g. in the pharmacy, the first chamber having valve means associated therewith to enable the aqueous component to be charged into the chamber when such dispensing takes place. Thereafter, the patient (or physician) can himself dispense the necessary unit doses from the charged pack containing both the lipid solution and the aqueous component.

As will be understood from the foregoing description, in putting the present invention into effect, the unit dose level of active material dispensed by each operation of the above-described pack may be varied widely depending on a number of factors, in particular:

1. The concentration of active material in the packed system;

2. The size of dosing valve employed;

3. The size of the discharge orifice; and

4. For continuous flow valves, the duration of action.

Furthermore, liposome size will be controlled in the main (we believe) by the pressure generated by the propellant or propellants and/or by the viscosity of the mixture sprayed. Generally speaking, the more vigorously the components are mixed e.g. by initial shaking or on discharge, the smaller will be the size of liposomes. However, the size of the discharge or dispensing orifice of the valve used to dispense the mixed components may also effect the size of liposomes produced, and preferably, such a discharge orifice has a size of from about 350 to about 450 micrometers (microns).

On the other hand, the above-mentioned parameters once having been fixed, the resultant dose level can readily be ascertained, as can also the degree of incorporation of any active material, and the size of liposomes produced.

As will be appreciated by those familiar with formulating active materials with carrier materials, the level of incorporation of active material within the liposomal carrier produced in the present invention may have a pronounced effect on the datum dose level chosen for the overall formulation. Accordingly, such a datum dose level will be a matter of formulating choice depending on the envisaged criteria to be met by any one particular formulation.

The invention further includes a composition for use in preparing a plurality of unit doses comprising a liposomal material, which composition comprises an aqueous component and a solution of a lipid component in an organic solvent, the composition including a propellant material or materials and, optionally, an active material as defined above, and the amount of water provided by the aqueous component being (as indicated above) sufficient to hydrate the lipid to permit formation of liposomes as such.

The invention still further includes a liposomal material or a liposomal composition when prepared as a spray by a method as described herein or by the use of a pack as described herein or a composition as defined above.

The following Examples illustrate the method, pack and composition of the invention.

In the specific Examples given the formulations include ammonium molybdate as an electron dense material, which was incorporated merely as a marker to facilitate detection of the liposomes and measurement of their diameter using an electron microscope. It will, of course, be understood by those familiar with pharmaceutical formulations that ammonium molybdate, which is moderately toxic, should not be part of an actual working formulation for administration to a patient. The omission of ammonium molybdate has no detrimental effect on the level of incorporation of active ingredient and, accordingly, in any of the exemplified formulations the molybdate marker can be omitted without detriment, and the given results can be obtained in its absence.

Also, while a variety of levels of active ingredient are exemplified, and while those in turn give a variety of levels of free and incorporated active ingredient per actuation, again it will be understood by the skilled formulator that the dose level and the kind of formulation chosen for any particular working pack will depend on the particular desired dosing regimen to be achieved, as well as any necessary or desired balance between unincorporated and incorporated active material.

Thus, active material can be dispensed at an overall dose level above or below (or even at) a known standard level, to provide any desired variation in effect, that variety of effect being enhanced by the degree of incorporation chosen.

## EXAMPLE 1

300 mg of egg lecithin and 4 mg of stanozolol were dissolved in 558 mg of ethyl alcohol (B.P.) and the thus-formed solution transferred to a standard 25 ml glass aerosol bottle (inhaler type). 9.0 ml of aqueous ammonium molybdate solution (0.5% w/w) and 3.0 g of dichlorodifluoromethane were added. The bottle was then sealed with a standard 60 microlitre metering valve and a standard actuator button with a 450 micron orifice diameter insert fitted. The bottle was shaken to produce an emulsion of the alcoholic and aqueous solutions and the thus-produced emulsion was sprayed as an aerosol. The resulting aerosol spray afforded unilamellar and multilamellar liposomes with diameters of between 30 nm and 1000 nm. Of the 20 micrograms of stanozolol released by each actuation of the metering valve, 13 micrograms were associated with the liposomes.

## EXAMPLES 2 to 6

The procedure of Example 1 was followed with the variations and results set out in the Table below.

| Example | Lecithin mg | Stanozolol mg | Ethyl alcohol mg | Aqueous ammonium molybdate solution (0.5% w/w) in ml | Valve dose size in micro- litres | Delivered drug per actuation in micrograms | Drug incorporated per actuation in micrograms |
|---|---|---|---|---|---|---|---|
| 2 | 600 | 8.0 | 1116 | 8.0 | 60 | 40 | 30 |
| 3 | 1200 | 16.0 | 2232 | 6.0 | 60 | 80 | 24 |
| 4 | 150 | 0 | 279 | 3.5 | 75 | 0 | 0 |
| 5 | 600 | 8.0 | 1116 | 8.0 | SC/F | 400 *(per second) | 190 *(per second) |
| 6** | 600 | 8.0 | 1116 | 8.0 | AC/F | N.D. | N.D. |

* 360 micron orifice diameter insert used in actuator button to control rate of release of mixture.

** In Example 6 the lipid and molybdate solutions were introduced into the inner, aluminium bag of an Alucompack container. The propellant was introduced into the outer cannister and the pack assembled with a continuous flow mixing valve. The propellant and combined solutions were therefore kept separate.

SC/F = Standard continuous flow valve.

AC/F = Continuous flow valve with integral mixing chamber as described in British Patent Applications Nos. 84-20850 and 84-20851, now published respectively as GB 2,145,107A and GB 2,148,401A.

N.D. = not determined.

EP 0 190 926 B1

Examples 7 to 9

Three aerosol systems were prepared containing bitolterol mesylate as follows:

A lipid phase was prepared comprising a solution of lecithin (300 mg/ml) and bitolterol mesylate (1mg/ml) in ethyl alcohol. Different amounts of the lipid phase (0.5, 1.0 and 3.0 ml respectively) were added to different amounts of 0.5% w/v ammonium molybdate solution in water (5.0, 5.0 and 3.0 ml respectively) in separate aerosol bottles. Propellant 12 i.e. dichlorodifluoromethane (2.6 g), was added to each bottle and the bottles capped, each with a standard 15 mm, 100 microlitre metering valve to provide three samples respectively having lipid phase:aqueous phase ratios of 1:10, 1:5, and 1:1 and respectively comprising about 92%, about 85% and about 54% by weight of water based on the total weight of lipid and aqueous phases. The bottles were shaken vigorously to mix the phases.

In the first two samples (1:10 and 1.5 lipid phase:aqueous phase ratios) a white emulsion was evident above the clear propellant phase. In the third sample a clear solution was seen above the clear propellant layer.

Electron microscopy of condensed bitolterol mesylate aerosols prepared from the samples by spraying revealed liposomes present in all three sprayed preparations but in limited numbers from the sample containing the 1:1 lipid phase:aqueous phase ratio. Dilution of the sample greatly increased the numbers of liposomes produced, which suggested that the third sample contained insufficient aqueous phase fully to hydrate the lecithin present in the lipid phase on spraying.

Results of the percentage drug incorporation determinations gave 59%, 60% and 10% by weight respectively for the three samples again suggesting that the liposomes were not fully formed in the third sample. All three samples produced a very fine aerosol spray when dispensed using actuator buttons (manufactured by Coster) fitted with "radial feed" inserts to provide mechanical break-up.

We are aware of European Patent Application No. 0,158,441 A2 published on 16th October, 1985, which describes and claims pro-liposome compositions. It is to be understood that those compositions while generally defined as ones which spontaneously form vesicles or liposomes in the presence of excess water, and comprising a uniform mixture of:-

a) at least one membrane lipid,

b) at least one water-miscible organic liquid which is a solvent for the lipid, and

c) up to 40% by weight of water,

the proportion by weight of a) to b) being from 40:1 to 1:20

are not themselves intended for the formation of liposomes as such, but only for the formation of proliposomes, which later may be converted to liposomes in the presence of excess water. Accordingly, the method and composition of the invention as defined in the claims appended hereto do not respectively lead to or include such pro-liposome compositions as therein described or defined.

## Claims

1.  A method for the preparation of liposomes, which method comprises preparing a solution of a lipid component in an organic solvent, combining the thus-formed solution with a second component comprising water, forming an intimate mixture from the combination, and passing a portion of the combination as said mixture under pressure through an orifice, thereby to produce an aerosol spray containing liposomes.

2.  A method according to claim 1, wherein an active material which is biologically-active and/or useful in the treatment or care of the human or animal body or of plants is included in the combination.

3.  A method according to claim 2, wherein the active material is dissolved in the organic solvent.

4.  A method according to claim 2 or claim 3, wherein the active material is:
    (i) therapeutically - or cosmetically-active;
    (ii) a biologically-active reagent;
    (iii) is one or more compounds having nutritional value;

or

(iv) a pesticide, herbicide or plant growth regulator.

5. A method according to any one of the preceding claims, wherein the lipid component comprises a phospholipid material and is chosen so as to provide a positively charged, a negatively charged or a neutral liposome, and preferably the phospholipid comprises a phosphatidyl choline, a phosphatidyl ethanolamine or a phosphatidyl serine, and the lipid component comprises either one or more of those materials alone or one or more of those materials together with cholesterol and/or at least one of dicetyl phosphate, phosphatidic acid or stearylamine.

6. A method according to any one of the preceding claims, wherein the aqueous component includes a buffering material.

7. A method according to any one of the preceding claims, wherein the spraying pressure is produced by at least one aerosol propellant.

8. A method according to any one of claims 2 to 7, wherein the mixture is sprayed in a manner which provides a metered dose of the said active material.

9. A pack for use in preparing a liposomal aerosol, which pack comprises at least a first chamber containing a solution of a lipid component in an organic solvent and an aqueous component, the first chamber and/or a second chamber including at least one propellant material, and the pack including an arrangement for dispensing through an orifice a mixture comprising the lipid solution and aqueous component as a spray under pressure developed by the propellant material or materials.

10. A pack according to claim 9, wherein the first chamber includes an active material which is biologically-active and/or useful in the treatment or care of the human or animal body or of plants.

11. A pack according to claim 10, wherein the dispensing arrangement is such that a metered dose of the said active material can be dispensed from the pack.

12. A pack according to any one of claims 9 to 11, wherein the lipid solution, aqueous component, propellant(s), and any said active material are as defined in any one of claims 3 to 7.

13. A composition for use in preparing a plurality of unit doses comprising a liposomal material, which composition comprises an aqueous component and a solution of a lipid component in an organic solvent, the composition optionally including a propellant material or materials and, optionally, an active material as defined in claim 2.

14. A composition according to claim 13, which comprises from about 45% to about 70% by weight of water, from about 2% to about 10% by weight of lipid, from about 4% to about 20% by weight of organic solvent, and optionally from about 20% to about 45% by weight of propellant.

15. A composition according to claim 13 or claim 14, wherein the weight ratios of ingredients are as follows:
    Water to lipid - from about 5:1 to about 30:1;
    Solvent to lipid - about 40:1 or lower e.g. 1.86:1;
    Water to solution - at least about 4:1;
    Water to propellant - from about 1.1:1 to about 20:1; and/or
    Propellant to solvent - from about 1.3:1 to about 12:1.

16. A composition according to any one of claims 13 to 15, wherein the lipid solution, aqueous component, propellant(s), and any said active material are as defined in any one of claims 3 to 7.

17. A modification of a pack for use in preparing a liposomal aerosol according to any one of claims 9 to 12, which pack comprises at least a first chamber containing a solution of a lipid component in an organic solvent, means to supply an aqueous component to the chamber, the first chamber, a second chamber and/or the aqueous component including at least one propellant material, and the pack including an arrangement for dispensing through an orifice a mixture of said solution and aqueous component as a spray under pressure developed by the propellant material or materials.

## Revendications

1. Procédé pour la préparation de liposomes, lequel procédé comprend les étapes consistant à préparer une solution d'un composant lipide dans un solvant organique, à combiner la solution ainsi formée avec un second composant comprenant de l'eau, à former un mélange intime à partir de la combinaison, et à passer une portion de la combinaison en tant que mélange sous pression à travers un orifice, produisant ainsi un spray aérosol contenant des liposomes.

2. Procédé selon la revendication 1, dans lequel une substance active qui est active biologiquement et/ou utile dans le traitement ou les soins du corps humain ou animal ou des plantes est compris dans la combinaison.

3. Procédé selon la revendication 2, dans lequel la substance active est dissous dans le solvant organique.

4. Procédé selon la revendication 2 ou la revendication 3, dans lequel la substance active est :
   1 active thérapeutiquement ou cosmétiquement ;
   2 un réactif actif biologiquement ;
   3 est un ou plusieurs composés ayant une valeur nutriticnnelle ;
   ou
   4 un pesticide, un herbicide ou un régulateur de croissance végétale.

5. Procédé selon l'une quelconque des revendications précédentes, dans lequel le composant lipide comprend une matière phospholipide et est choisi de façon à fournir un liposome positivement chargé, un liposome négativement chargé ou un liposome neutre, et de préférence le phospholipide comprend une phosphatidyle choline, une phosphatidyle éthanolamine ou une phosphatidyle sérine, et le composant lipide comprend soit une ou plusieurs de ces matières seules ou une ou plusieurs de ces matières conjointement avec le cholestérol et/ou au moins une des substances suivantes : dicétyle phosphate, acide phosphatidique ou stéarylamine.

6. Procédé selon l'une quelconque des revendications précédentes, dans lequel le composant aqueux comprend une matière tampon.

7. Procédé selon l'une quelconque des revendications précédentes, dans lequel la pression de pulvérisation est produite par au moins un agent propulseur d'aérosol.

8. Procédé selon l'une quelconque des revendications 2 à 7, dans lequel le mélange est pulvérisé d'une manière qui fournit une dose mesurée de la substance active.

9. Récipient destiné à l'utilisation dans la préparation d'un aérosol liposomal, lequel récipient comprend au moins une première chambre contenant une solution d'un composant lipide dans un solvant organique et un composant aqueux, la première chambre et/ou une seconde chambre comprenant au moins un agent expulseur, et le récipient comprenant un agencement destiné à délivrer à travers un orifice un mélange comprenant la solution lipide et le composant aqueux sous forme d'une pulvérisation sous la pression développée par l'agent propulseur ou les matières.

10. Récipient selon la revendication 9, dans lequel la première chambre comprend une substance active qui est active biologiquement et/ou utile dans le traitement ou les soins au corps humain ou animal ou aux plantes.

11. Récipient selon la revendication 10, dans lequel le dispositif de distribution est de telle manière qu'une dose mesurée de la substance active peut être délivrée dans le récipient.

12. Récipient selon l'une quelconque des revendications 9 à 11, dans lequel la solution lipide, le composant aqueux, les agents propulseurs, et toutes substances actives sont définis dans l'une quelconque des revendications 3 à 7.

13. Composition destinée à l'utilisation dans la préparation de plusieurs dosages unitaires comprenant une matière liposomale, laquelle composition comprend un composant aqueux et une solution d'un composant lipide dans un solvant organique, la composition comprenant un ou plusieurs agents propulseurs et optionnellement, une substance active telle que définie dans la revendication 2.

14. Composition selon la revendication 13, qui comprend d'environ 45% à environ 70% en poids d'eau, d'environ 2% à environ 10% en poids de lipide, d'environ 4% à environ 20% en poids de solvant organique, et optionnellement d'environ 20% à environ 45% en poids d'agent propulseur.

15. Composition selon la revendication 13 ou la revendication 14, dans laquelle les rapports pondéraux des ingrédients sont les suivants :
    Eau par rapport aux lipides d'environ 5:1 à d'environ 30:1 ;
    Solvant aux lipides - environ 40:1 ou inférieur, par exemple 1,86:1 ;
    Eau par rapport à la solution - au moins environ 4:1 ;
    Eau par rapport à l'agent propulseur - d'environ 1,1:1 jusqu'à environ 20:1 ; et/ou
    Agent propulseur par rapport au solvant - d'environ 1,3:1 jusqu'à environ 12:1.

16. Composition selon l'une quelconque des revendications 13 à 15, dans laquelle la solution lipide, composant aqueux, les agents propulseurs et toutes substances actives sont telles que définies dans l'une quelconque des revendications 5 à 7.

17. Modification d'un récipient destiné à l'utilisation dans la préparation d'un aérosol liposomal selon l'une quelconque des revendications 9 à 12, lequel récipient comprend au moins une première chambre contenant une solution de composants lipides dans un solvant organique, des moyens pour amener un composant aqueux à la chambre, la première chambre, une seconde chambre et/ou le composant aqueux comprenant au moins un agent propulseur, et le récipient contenant un agencement destiné à délivrer par un orifice un mélange de la solution et du composant aqueux sous forme de spray sous la pression développée par l'agent ou les agents propulseurs.

## Ansprüche

1. Verfahren zur Herstellung von Liposomen, das die Herstellung einer Lösung einer Lipidkomponente in einem organischen Lösungsmittel, Kombinieren der so gebildeten Lösung mit einer zweiten Komponente, die Wasser umfaßt, Bilden einer innigen Mischung aus dieser Kombination und Hindurchführen eines Teils der Kombination als derartige Mischung unter Druck durch eine Öffnung, wobei ein Aerosol-Spray erzeugt wird, das Liposome enthält, beinhaltet.

2. Verfahren nach Anspruch 1, bei dem ein Wirkstoff, der biologisch aktiv und/oder bei der Behandlung oder Pflege des menschlichen oder tierischen Körpers oder von Pflanzen nützlich ist, mit in die Kombination aufgenommen wird.

3. Verfahren nach Anspruch 2, bei dem der Wirkstoff in dem organischen Lösungsmittel gelöst ist.

4. Verfahren nach Anspruch 2 oder 3, bei dem der Wirkstoff:
    (i) therapeutisch- oder kosmetisch wirksam,
    (ii) ein biologisch aktives Reagens,
    (iii) eine oder mehrere Verbindungen mit Nährwert ist,
    oder
    (iv) ein Pestizid, Herbizid oder Pflanzenwachstumsregulator ist.

5. Verfahren nach einem der vorhergehenden Ansprüche, bei dem die Lipidkomponente ein Phospholipid umfaßt und so gewählt wird, daß es ein positiv geladenes, negativ geladenes oder neutrales Liposom liefert, und das Phospholipid vorzugsweise ein Phosphatidylcholin, Phosphatidylethanolamin oder Phosphatidylserin umfaßt, und die Lipidkomponente entweder einer oder mehrere dieser Stoffe alleine

13

oder einer oder mehrere dieser Stoffe zusammen mit Cholesterin und/oder mindestens einer der Verbindungen Dicetylphosphat, Phosphatidsäure oder Stearylamin umfaßt.

6. Verfahren nach einem der vorhergehenden Ansprüche, bei dem die wäßrige Komponente eine Puffersubstanz umfaßt.

7. Verfahren nach einem der vorhergehenden Ansprüche, bei dem der Sprühdruck durch mindestens ein Aerosol-Treibmittel erzeugt wird.

8. Verfahren nach einem der Ansprüche 2 bis 7, bei dem die Mischung in einer Weise versprüht wird, die eine abgemessene Dosis des Wirkstoffs liefert.

9. Behälter zur Verwendung bei der Herstellung eines Liposom-Aerosols, der mindestens eine erste Kammer umfaßt, die eine Lösung einer Lipidkomponente in einem organischen Lösungsmittel und eine wäßrige Komponente enthält, wobei die erste Kammer und/oder eine zweite Kammer mindestens eine Treibmittelsubstanz einschließt und der Behälter eine Einrichtung zum Abgeben einer Mischung, die die Lipidlösung und die wäßrige Komponente umfaßt, als Spray unter Druck aufweist, der durch die Treibmittelsubstanz oder -substanzen entwickelt wurde.

10. Behälter nach Anspruch 9, worin die erste Kammer einen Wirkstoff einschließt, der biologisch aktiv und/oder bei der Behandldung oder Pflege des menschlichen oder tierischen Körpers oder von Pflanzen nützlich ist.

11. Behälter nach Anspruch 10, worin die Abgabeeinrichtung derart ist, daß eine abgemessene Dosis des Wirkstoffs aus dem Behälter abgegeben werden kann.

12. Behälter nach einem der Ansprüche 9 bis 11, worin die Lipidlösung, die wäßrige Komponente, das (die) Treibmittel und irgendeiner der Wirkstoffe wie in einem der Ansprüche 3 bis 7 definiert sind.

13. Zusammensetzung zur Verwendung bei der Herstellung einer Vielzahl von liposomales Material enthaltenden Dosierungseinheiten, wobei die Zusammensetzung eine wäßrige Komponente und eine Lösung einer Lipidkomponente in einem organischen Lösungsmittel umfaßt, wobei die Zusammensetzung eine Treibmittelsubstanz oder -substanzen und gegebenenfalls einen in Anspruch 2 definierten Wirkstoff enthält.

14. Zusammensetzung nach Anspruch 13, die etwa 45 bis etwa 70 Gew.-% Wasser, etwa 2 bis etwa 10 Gew.-% Lipid, etwa 4 bis etwa 20 Gew.-% organisches Lösungsmittel und gegebenenfalls etwa 20 bis etwa 45 Gew.-% Treibmittel umfaßt.

15. Zusammensetzung nach Anspruch 13 oder 14, worin die Gewichtsverhältnisse der Bestandteile wie folgt sind:

| | |
|---|---|
| Wasser zu Lipid - | etwa 5:1 bis etwa 30:1; |
| Lösungsmittel zu Lipid - | etwa 40:1 oder weniger, z.B. 1,86:1; |
| Wasser zu Lösung - | mindestens etwa 4:1; |
| Wasser zu Treibmittel - | etwa 1,1:1 bis etwa 20:1; und/oder |
| Treibmittel zu Lösungsmittel - | etwa 1,3:1 bis etwa 12:1. |

16. Zusammensetzung nach einem der Ansprüche 13 bis 15, worin die Lipidlösung, die wäßrige Komponente, das (die) Treibmittel und jeder der Wirkstoffe wie in einem der Ansprüche 3 bis 7 definiert sind.

17. Variante eines Behälters zur Verwendung bei der Herstellung eines Liposom-Aerosols nach einem der Ansprüche 9 bis 12, wobei der Behälter mindestens eine erste Kammer, die eine Lösung einer Lipidkomponente in einem organischen Lösungsmittel enthält, und Mittel zum Zuführen einer wäßrigen Komponente zu der Kammer umfaßt, wobei die erste Kammer, eine zweite Kammer und/oder die wäßrige Komponente mindestens eine Treibmittelsubstanz enthalten und die Behälter eine Vorrichtung zur Abgabe einer Mischung der Lösung und der wäßrigen Komponente durch eine Öffnung als Spray unter Druck aufweisen, der durch die Treibmittelsubstanz oder - substanzen entwickelt wurde.